# EUROPEAN PATENT APPLICATION

(11) **EP 2 407 554 A1**
(43) Date of publication of application: **18.01.2012**
(21) Application number: 10382193.0
(22) Date of filing: 14.07.2010
(51) Int. Cl.: C12Q 1/68

(54) **METHODS AND KITS FOR THE DIAGNOSIS OF PROSTATE CANCER**

(71) Applicant: Fundacio Institut de Recerca de l'Hospital Universitari Vall d'Hebron, 08035 Barcelona (ES)
(72) Inventor: DOLL, Andreas, 08035 Barcelona (ES); RIGAU RESINA, Marina, 08035 Barcelona (ES); ABAL POSADA, Miguel, 08035 Barcelona (ES); MOROTE ROBLES, Juan, 08035 Barcelona (ES); REVENTÓS PUIGJANER, Jaume, 08035 Barcelona (ES)
(74) Representative: ABG Patentes, S.L.

(57) **Abstract**

The present invention relates to the use of PSGR, a member of the G-protein-couplcd olfactory receptor family which is over-expressed in prostate cancer tissue, as a marker suitable for an urine-based diagnostic test for prostate cancer which require only typical prostate manipulation and sample acquisition scenarios avoiding invasive tissue collection.

## Description

### FIELD OF THE INVENTION

The invention fall within the field of diagnosis and, more specifically, in the field of diagnosis of prostate cancer by means of using a marker gene the expression of which is over-expressed in urine samples from prostate cancer patients.

### BACKGROUND OF THE INVENTION

Prostate cancer (PCa) has transformed into the most common type of cancer in the Western male population, where it is responsible for more male deaths than any other cancer, except lung cancer. Even though the introduction of the prostate specific antigen (PSA) test in the late 80s of the past century has led to a dramatic increase its detection (Jemal A. et al. Cancer statistics, 2007,57:43-66), the risk of developing this type of cancer during a lifetime is estimated at one in six men, and the risk of death due to metastatic PCa is 1 in 30.

The diagnostic tools for detecting PCa can be separated into those that screen for the disease, such as PSA and digital rectal exams (DRE), and the decisive diagnosis set of transrectal ultrasound guided prostate biopsies (TRUS).

One of the limitations of serum PSA as a tumor marker is its lack of specificity, which results in a high rate of negative biopsies. There is a substantial overlap in the serum PSA values of men with benign prostatic hyperplasia (BPH) and those with PCa. Elevated PSA levels can also be attributed to other factors such as prostatitis, prostate irritation, and recent ejaculation (Pannek J y Martin AW, Oncology, 11:1273-8 y Loeb, S. 2009, Urol. Oncol.27:64-6). As a consequence of the current screening parameters, around 2/3 of the approximately 1,300,000 biopsies made yearly in the United States (390,000 in Europe) are unnecessary (Catalona WJ. et al., 1991, N. Engl. J. Med. 324:1156-61 y Makinen, T. et al. 2004, Clin Cancer Res 10:2231-6). In contrast, the false positive rate of a biopsy is about zero, although the false negative rate in the first biopsy may oscillate between 12-32% (Cervera Deval J. et al., 2004, Actas Urol. Esp. 28:666-71 and Raber M. et al,. 2000, Arch. Ital. Urol. Androl. 72:197-9). Consequently, large populations of men with chronically elevated serum PSA undergo repeated biopsies to rule out PCa (Pannek J y Partin AW, Oncology, 11:1273-8 y Loeb, S. 2009, Urol. Oncol.27:64-6).

Various attempts have been made to overcome the limitations of PSA screening, including the use of Ratio PSA, age-adjusted PSA cut-off points, free-PSA, PSA-density, PSA-velocity, PSA-slope and PSA-doubling time, and (-2) proPSA (Mikolajczyk SD. et al., 2004, Clin Biochem., 37:519-528; Sokoll L.J. et al., 2008, J. Urol 2008:180:539-543 and Morote. J. et al., 1997, J. Urol. 158:502-504). All have been proposed as a means of improving serum PSA specificity in the detection of PCa. Nevertheless, there has been no evidence to suggest that any of these testing strategies improves health outcomes (Harris R and Lohr KN; 2002, Ann. Intern. Med. 137:917-929).

Yu et al. (Prostate Cancer Prostatic Dis., 2006, 9:56-61) have described that the mRNA of the prostate - specific G protein coupled receptor (PSGR) gene is over-expressed in prostatic epithelial cells of malignant glands compared to benign epithelial prostate cells thus suggesting that this gene could be used as a marker for prostate cancer. Weng et al. (Int J Cancer., 2005, 113:811-8) have described that the expression of PSGR is significantly increased in human prostate intraepithelial neoplasia and prostate tumors compared to normal and benign prostatic hyperplasia tissues, thus suggesting that PSGR may have a potential for early prostate cancer and diagnosis.

However, these markers are detected in prostate tissue samples and thus, require invasive procedures for their determination.

There is an urgent need to detect prostate cancer at an early stage using non-invasive procedures.

### SUMMARY OF THE INVENTION

The authors of the present invention have found that PSGR, a member of the G-protein-coupled olfactory receptor family, shows a highly prostate tissue-specific expression and tumor associated over-expression restricted to prostate epithelial cells and that said over-expression can be detected in urine samples, thus making PSGR a urinary marker for prostate cancer. As prostate cells can be detected in the urine of men with prostate cancer, this finding allows the development of urine-based diagnostic tests which require only typical prostate manipulation and sample acquisition scenarios avoiding invasive tissue collection approaches.

Thus, in a first aspect, the invention relates to a method for the diagnosis of prostate cancer in a subject comprising
(i) determining the levels of PSGR mRNA in a biofluid from said subject and
(ii) comparing the levels obtained in step (i) with reference values of said mRNA
wherein increased levels of PSGR mRNA with respect to the reference value is indicative that the subject suffers prostate cancer.

In another aspect, the invention relates to a method for assessing or monitoring the response to a therapy in a subject having prostate cancer (PCa) which comprises
(i) determining the expression levels of the PSGR mRNA in an biofluid sample from said subject after administering said therapy,
(ii) comparing the levels obtained in step (i) with reference value for said mRNA obtained prior to administration of the therapy
wherein a significant decrease in the PSGR mRNA levels for said gene in the biofluid after the administration of said therapy in comparison with the level of expression of each of said genes prior to the administration of the therapy in the subject sample is indicative that the therapy administered to the subject is efficacious
or wherein a significant increase or lack of change in the PSGR mRNA levels for said gene in the biofluid after the administration of said therapy in comparison with the level of expression of each of said genes prior to the administration of the therapy in the subject sample is indicative that the therapy administered to the subject is inefficacious.

In another aspect, the invention relates to a method for monitoring the progression of prostate cancer (PCa) in a subject comprising
(i) determining the expression levels of the PSGR mRNA in an urine sample from said subject,
(ii) comparing the levels obtained in step (i) with reference value for said mRNA obtained from the same subject at an earlier time point of the disease
wherein a significant decrease in the PSGR mRNA for said gene in the biofluid in comparison with the level of expression of said mRNA at the earlier time point is indicative that the prostate cancer shows a good progression or
wherein a significant increase or a lack of change in the PSGR mRNA for said gene in the biofluid in comparison with the level of expression of said mRNA at the earlier time point is indicative that the prostate cancer shows a bad progression.

### BRIEF DESCRIPTION OF THE DRAWINGS

**Figure 1****.** Characterization of the urine based biomarker of prostate cancer (box and-whisker plots). (A) Relative level of PSGR. (B) Relative level of PCA3.
**Figure 2****.** Receiver-operating-characteristic (ROC) curves for PSGR and PCA3 in men with detected prostate cancer and men with no cancer detected at biopsy (n=215). ROC curve for PSGR (light grey line), ROC curve for PCA3 (dark grey line), MultiROC curve for PSGR in combination with PCA3 (black line) and ROC curve for PSA (dashed black line).

### DETAILED DESCRIPTION OF THE INVENTION

### Diagnostic method of the invention

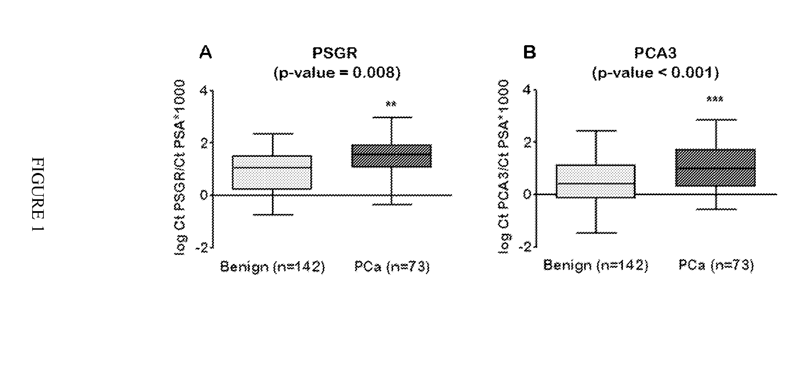

The authors of the present invention have found that PSGR, a member of the G-protein-coupled olfactory receptor family, shows a highly prostate tissue-specific expression and tumor associated over-expression restricted to prostate epithelial cells and that said over-expression can be detected in urine samples, thus making PSGR a urinary marker for prostate cancer. As shown in example 1 and in figure 1 of the present invention, PSGR expression levels in urine was a significant predictor (p=0.008) of patients with PCa and patients with negative needle biopsies. Moreover, as prostate cells can be detected in the urine of men with prostate cancer, this finding allows the development of urine-based diagnostic tests which requires only typical prostate massage and urine sample acquisition.

Thus, in a first aspect, the invention relates to a method for the diagnosis of prostate cancer in a subject comprising
(i) determining the levels of PSGR mRNA in a biofluid from said subject and
(ii) comparing the levels obtained in step (i) with reference values said mRNA wherein increased levels of PSGR mRNA in urine with respect to the reference value is indicative that the subject suffers prostate cancer.

As it is used herein, the expression "method for the diagnosis" relates to a method that may essentially consist of the previously mentioned steps or may include additional steps. However, it must be understood that the method, in a preferred embodiment, is a method that is carried out *in vitro,* i.e., it is not carried out in the human or animal body. Diagnosing as used herein relates to evaluating the probability according to which a subject suffers from a disease. As it will be understood by persons skilled in the art, such evaluation, although it is preferred that it is, normally may not be correct for 100% of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley & Sons, New York 1983. The preferred confidence intervals are of at least 50%, at least 60%, at least 70%, at least 80%, at least 90%, at least 95%, preferably of at least 99%. The p values are preferably 0.2, 0.1, 0.05, preferably 0.01. The method for the diagnosis of the invention allows assessing whether a subject is afflicted with PCa.

A "subject", as used herein, refers to a any animal classified as mammal and includes but is not limited to domestic and farm animals, primates and humans, for example, human beings, non-human primates, cows, horses, pigs, sheep, goats, dogs, cats, or rodents. Preferably, the subject is a male human being of any age or race.

In a preferred embodiment, the subject wherein the determination is carried out shows PSA levels which are higher than 4 ng/ml. In a still more preferred embodiment, the method of the invention is carried out in a subject showing PSA levels lower than 10 ng/ml and, more preferably, between 4 and 10 ng/ml. In another embodiment, the method of the invention is carried out in subjects which have not been subjected to prostate biopsy.

In a preferred embodiment, the subject wherein the method of the invention is applied refers to a patient suffering isolated high-grade prostate intraepithelial neoplasia.

The term "high-grade prostate intraepithelial neoplasia" or "HG-PIN", as used herein, refers to a condition associated with high risk of prostate cancer and also known as dysplasia, intraductal dysplasia, large acinar atypical hyperplasia, atypical primary hyperplasia, hyperplasia with malignant change, marked atypia, and duct-acinar dysplasia. HG-PIN may be characterized by a high nuclear/cytoplasmic ratio, hyperchromasia, coarsely granular chromatin, absence of nucleoli, isolated cells and cellular and nuclear pleomorphism. Methods used for diagnosis of PIN are known in the art and include, but are not limited to, needle biopsy and measurement of expression of ezrin, a cytoskeleton linker protein that is actively involved in regulating the growth and metastatic capacity of cancer cells {see, Pang et ai, Urology. 2004 Mar; 63(3):609-12, and U.S. Patent No. 6,054,320 provides kits for identification of PIN.

Thus, patients diagnosed with HG-PIN are particularly suitable candidates for the method of the invention as they are at high risk of developing prostate cancer as a result of malignant conversion of the HG-PIN.

The term "prostate cancer" refers to any cancer of the prostate gland in which cells of the prostate mutate and begin to multiply out of control irrespective of the stage of the cancer. The extent to which prostate cancer has progressed in a patient is assessed taking into account clinical and histopathological information. The stage of cancer is classified based on tumour size (T), whether there is lymph node involvement (N), the presence of metastasis (M), and the tumour grading (G). A tumour classed as T1 is confined to the prostate gland and too small to be felt by digital rectal examination. T1 further includes T1a (fewer than 5% cancerous cells in tissue sample) and T1b (more than 5%) subdivisions. T1c indicates the patient has an elevated Prostate Specific Antigen (PSA; see definition later). If the tumour is large enough to be felt during a digital rectal examination, it is classified as T2. T2a means only one side of the prostate gland (left or right) is involved; T2b means both sides have a tumour(s). T2 is commonly termed "localized cancer". If the cancer is T3, it has spread to the connected tissue near the prostate (T3a) or the seminal vesicles (T3b). T4 indicates cancer spread to tissue next to the prostate, for example the bladder sphincter, rectum or pelvis wall. The prostate cancer may also spread into the regional lymph nodes of the pelvis and this is assessed as N1 stage of prostate cancer. These stages of T3, T4 and N1 are collectively termed "locally advanced" or regional cancer. If the cancer has spread to distant sites, such as the bone, it is said to be "metastasized" or at the M1 stage. Prostate cancer that has spread to distant lymph nodes is categorized as M1 a while that which has spread to bone is M1b and that which has spread to organs such as liver or brain is assessed as M1c. Left untreated, prostate cancer almost universally metastasizes to bone.

In a first step, the diagnostic method of the invention comprises the determination of the PSGR mRNA levels in a biofluid isolated from the subject to be diganosed.

The expression "expression levels of a gene" as used in accordance with the method of the invention relates to the degree of gene expression of one or more genes. Typically, the expression levels of a given gene can be determined by measuring the RNA expression level, wherein the term "RNA expression level" refers to a determined level of the converted DNA gene sequence information into transcribed RNA, the initial unspliced RNA transcript or the mature mRNA.

RNA expression can be monitored by measuring the levels of either the entire RNA of the gene or subsequences. Virtually any method for detecting the presence of and for quantifying the level of a gene can be used within the frame of the instant invention in order to detect and quantify the levels of mRNA encoded by the biomarker genes. By way of a non-limiting illustration, in a particular embodiment, for measuring the amount of a particular RNA in a sample, methods known to one of ordinary skill in the art can be used to extract and quantify transcribed RNA from a sample with respect to the biomarker genes (PSGR). Please, see WO2008/121132 and WO 98/24935 herein incorporated by reference for RNA analysis protocols. Briefly, RNA is extracted from a sample such as any tissue, body fluid, cell (e.g., circulating tumor cell), etc. For example, cells may be lysed and RNA eluted in a suitable solution in which to conduct a DNAse reaction. Subsequent to RNA extraction, first strand synthesis may be performed using a reverse transcriptase. Gene amplification, more specifically quantitative PCR assays, can then be conducted and the gene of interest calibrated against an internal marker such as, e.g., 18S rRNA, although any other endogenous marker can also be used, such as 28S-25S rRNA and 55 rRNA. Samples are measured in multiple replicates, for example, 3 replicates. In an embodiment of the invention, qPCR is performed using amplification, reporting agents and instruments such as those supplied commercially by Applied Biosystems. Given a defined efficiency of amplification of target transcripts, the point (e.g., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e.g., a reference standard curve) or normalized to a standard with limited variability can be used to quantify the number of target templates in an unknown sample.

Although not limited to amplification methods, quantitative gene expression techniques may use amplification of the target transcript. Alternatively, or in combination with amplification of the target transcript, quantification of the reporter signal for an internal marker generated by the exponential increase of amplified product may also be used. Amplification of the target template may be accomplished by isothermic gene amplification strategies or by gene amplification by thermal cycling such as PCR.

Specific RNAs are amplified using message specific primers or random primers. The specific primers can be synthesized from data obtained from public databases (e.g., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples [see, for example, RT PCR, Chapter 15 in RNA Methodologies. A Laboratory Guide for Isolation and Characterization. 2nd edition, 1998, Robert E. Farrell, Jr., Ed., Academic Press; or Chapter 22 pp.143-151, RNA Isolation and Characterization Protocols. Methods in Molecular Biology, Volume 86, 1998, R. Rapley and D. L. Manning Eds., Human Press, or Chapter 14 Statistical refinement of primer design parameters; or Chapter 5, pp.55-72, PCR Applications: protocols for functional genomics, M.A.Innis, D.H. Gelfand and J.J. Sninsky, Eds., 1999, Academic Press]. Amplifications are carried out in either isothermic conditions or using a thermal cycler [for example, a ABI 9600 or 9700 or 7900 obtained from Applied Biosystems]. Amplified nucleic acids are detected using fluorescent-tagged detection oligonucleotide probes [see, for example, Taqman™ PCR Reagent Kit, Protocol, part number 402823, Revision A, 1996, Applied Biosystems] that are identified and synthesized from publicly known databases as described for the amplification primers.

For example, without limitation, amplified cDNA is detected and quantified using a suitable detection system, e.g., the ABI Prism® 7900 Sequence Detection System (Applied Biosystems). Amounts of specific RNAs contained in the test sample can be related to the relative quantity of fluorescence observed (see, for example, Advances in Quantitative PCR Technology: 5' Nuclease Assays, Y.S. lie and CJ. Petropolus, Current Opinion in Biotechnology, 1998, 9:43-48, or Rapid Thermal Cycling and PCR Kinetics, pp. 211-229, chapter 14 in PCR applications: protocols for functional genomics, M. A. Innis, D.H. GelfandandXJ. Sninsky, Eds., 1999; Academic Press).

In a particular embodiment, quantitative PCR (qPCR) is used to detect and quantify the levels of expression of the PSGR.gene. Conventional methods of quantifying the genes expression levels can be found, for example, in Sambrook et al., 2001 "Molecular cloning: to Laboratory Manual", 3rd ed., Cold Spring Harbor Laboratory Press, N.Y., Vol. 1-3.

The term "PSGR", as used herein, refers to the prostate-specific G-protein coupled receptor), also known as OR51E2 (olfactory receptor, family 51, subfamily E, member 2, NM_030774), which is a member of G-protein coupled OR family that is highly prostate tissue-specific and its tumor-associated overexpression.

The term variant, as used herein, refers to polynucleotides resulting from the deletion, insertion or substitution of one or more nucleotides from the PSGR polynucleotide and which show a correlation between their expression levels and the presence of prostate cancer. Variants of the PSGR include, without limitation, polynucleotides showing a degree of identify of at least 70%, advantageously of at least 75%, typically of at least 80%, preferably of at least 85%, more preferably of at least 90%, still more preferably of at least 95%, 97%, 98% or 99%, with respect to the PSGR mRNA. The degree of identity between two amino acid sequences can be determined by conventional methods, for example, by means of standard sequence alignment algorithms known in the state of the art, such as, for example, BLAST [Altschul S.F. et al. Basic local alignment search tool. J Mol Biol. 1990 Oct 5; 215(3):403-10].

The term "RNA expression level" refers to a determined level of the converted DNA gene sequence information into transcribed RNA, the initial unspliced RNA transcript or the mature mRNA. RNA expression can be monitored by measuring the levels of either the entire RNA of the gene or subsequences. Virtually any method for detecting the presence of and for quantifying the level of a gene can be used within the frame of the instant invention in order to detect and quantify the levels of mRNA encoded by the biomarker genes. By way of a non-limiting illustration, in a particular embodiment, for measuring the amount of a particular RNA in a sample, methods known to one of ordinary skill in the art can be used to extract and quantify transcribed RNA from a sample with respect to the biomarker genes (PSGR). Please, see W02008/121132 and WO 98/24935 herein incorporated by reference for RNA analysis protocols. Briefly, RNA is extracted from a sample such as any tissue, body fluid, cell (e.g., circulating tumor cell), etc. For example, cells may be lysed and RNA eluted in a suitable solution in which to conduct a DNAse reaction. Subsequent to RNA extraction, first strand synthesis may be performed using a reverse transcriptase. Gene amplification, more specifically quantitative PCR assays, can then be conducted and the gene of interest calibrated against an internal marker such as, e.g., 18S rRNA, although any other endogenous marker can also be used, such as 28S-25S rRNA and 5S rRNA. Samples are measured in multiple replicates, for example, 3 replicates. In an embodiment of the invention, qPCR is performed using amplification, reporting agents and instruments such as those supplied commercially by Applied Biosystems. Given a defined efficiency of amplification of target transcripts, the point (e.g., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e.g., a reference standard curve) or normalized to a standard with limited variability can be used to quantify the number of target templates in an unknown sample.

Although not limited to amplification methods, quantitative gene expression techniques may use amplification of the target transcript. Alternatively, or in combination with amplification of the target transcript, quantification of the reporter signal for an internal marker generated by the exponential increase of amplified product may also be used. Amplification of the target template may be accomplished by isothermic gene amplification strategies or by gene amplification by thermal cycling such as PCR.

Specific RNAs are amplified using message specific primers or random primers. The specific primers can be synthesized from data obtained from public databases (e.g., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples [see, for example, RT PCR, Chapter 15 in RNA Methodologies. A Laboratory Guide for Isolation and Characterization. 2nd edition, 1998, Robert E. Farrell, Jr., Ed., Academic Press; or Chapter 22 pp.143-151, RNA Isolation and Characterization Protocols. Methods in Molecular Biology, Volume 86, 1998, R. Rapley and D. L. Manning Eds., Human Press, or Chapter 14 Statistical refinement of primer design parameters; or Chapter 5, pp.55-72, PCR Applications: protocols for functional genomics, M.A.Innis, D.H. Gelfand and J.J. Sninsky, Eds., 1999, Academic Press]. Amplifications are carried out in either isothermic conditions or using a thermal cycler [for example, a ABI 9600 or 9700 or 7900 obtained from Applied Biosystems]. Amplified nucleic acids are detected using fluorescent-tagged detection oligonucleotide probes [see, for example, Taqman™ PCR Reagent Kit, Protocol, part number 402823, Revision A, 1996, Applied Biosystems] that are identified and synthesized from publicly known databases as described for the amplification primers.

For example, without limitation, amplified cDNA is detected and quantified using a suitable detection system, e.g., the ABI Prism® 7900 Sequence Detection System (Applied Biosystems). Amounts of specific RNAs contained in the test sample can be related to the relative quantity of fluorescence observed (see, for example, Advances in Quantitative PCR Technology: 5' Nuclease Assays, Y.S. lie and CJ. Petropolus, Current Opinion in Biotechnology, 1998, 9:43-48, or Rapid Thermal Cycling and PCR Kinetics, pp. 211-229, chapter 14 in PCR applications: protocols for functional genomics, M. A. Innis, D.H. GelfandandXJ. Sninsky, Eds., 1999; Academic Press).

In a particular embodiment, quantitative PCR (qPCR) is used to detect and quantify the levels of expression of the PSGR gene. Conventional methods of quantifying the genes expression levels can be found, for example, in Sambrook et al., 2001 "Molecular cloning: to Laboratory Manual", 3rd ed., Cold Spring Harbor Laboratory Press, N.Y., Vol. 1-3.

In the present invention, the term "biofluid" refers to a biological liquid of a human or animal that can be excreted (such as urine or sweat), secreted (such as breast milk or bile), obtained with a medical device, for example, a needle (such as blood, plasma, serum, tears, buffer, swab extract, cell lysate, sputum, stool, saliva, bone marrow, wash or aspirate (bronchial, nasal, tracheal), cerebrospinal fluid), or develop as a result of a pathological process (e.g., blister or cyst fluid) taken from the subject, by any suitable means, e.g., venipuncture, excretion, ejaculation, massage, biopsy, needle aspirate, lavage sample, scraping, surgical incision or intervention or other means known in the art.

The biological sample should contain any biological material suitable for detecting the desired biomarker; thus, said sample should, advantageously comprise cell material, e.g., circulating tumor cells, from the subject. Thus, the sample, which should provide a source of nucleic acid from prostate tissue or cells, can be isolated from any suitable tissue or biological fluid, such as, for example, prostate cells, prostate tissue, prostatic fluid, urine, ejaculate, semen, etc. In practice, since prostate cells can be detected in the urine of men with PCa, the use of fluids such as urine is recommended. In a particular embodiment, the samples used for the determination of the biomarker according to the present invention are preferably urine samples obtained after a prostate massage (e.g., DRM) or after prostate biopsy (post-biopsy) or by spontaneous miction, preferably, urine samples obtained after a prostate massage (e.g., DRM) since it appears to be the best compromise between a minimal invasive technique accepted by a wider range of the male population and the possibility to obtain enough cells for a correct diagnosis. In another embodiment, the samples used for the determination of the biomarker according to the present invention are samples obtained by expressed prostatic secretions or prostatectomy (useful as negative controls).

The second step of the diagnostic method of the invention comprises comparing the levels obtained in step (i) with a reference value of said mRNA.

A "reference value", as used in the context of this inventive aspect, means a sample obtained from a pool of healthy subjects which are not afflicted with PCa. The suitable reference expression levels for the PSGR gene can be determined by measuring the expression levels of said gens in several suitable subjects, and such reference levels can be adjusted to specific subject populations. In a preferred embodiment, the reference sample is obtained from several healthy subjects or from subjects without prior history of PCa. The person skilled in the art will appreciate that the type of reference sample can vary depending on the specific method to be performed. Thus, if a method of diagnosis of the disease is to be carried out, a pool of non-tumor prostate tissue or non-tumor prostate cells samples from subjects that do not have a history of PCa can be used as reference. However, in the event that the method of the invention is aimed at determining the effect of a therapy in a patient, the reference sample is preferably a sample obtained from said patient prior to receive said treatment or at different time periods during a course of treatment. The expression profile of the PSGR gene in the reference sample can, preferably, be generated from a population of two or more subjects. The population, for example, can comprise 3, 4, 5, 10, 15, 20, 30, 40, 50 or more subjects. Furthermore, the expression profile of the PSGR gene in the reference sample and in the sample of the subject that is going to be diagnosed according to the methods of the present invention can be generated from the same subject, provided that the profiles to be assayed and the reference profile are generated from biological samples taken at different times and are compared to one another. For example, a sample of a subject can be obtained at the beginning of a study period. A reference biomarker profile from this sample can then be compared with the biomarker profiles generated from subsequent samples of the same subject.

Once the expression levels of the biomarker genes in the subject sample and the expression levels of said genes in a reference sample have been determined, it is necessary to identify if there is an increase in the expression of each one of said genes in the subject sample in comparison with the expression levels thereof in the reference sample. An increase in the expression of a gene in a sample of the subject under study is considered a "significant increase" with respect to the normal or reference level when the expression level in the subject sample increases with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

The authors of the present invention have observed that the diagnostic capacity of the mRNA levels of PSGR in urine can be further improved by combining said expression levels with expression levels of a second marker, PCA3. For instance, as shown in the example of the present invention, the combined marker model (PSGRvPCA3) allows to diagnose the presence of prostate cancer with a sensitivity of 0.77 [0.65-0.85] and a specificity of 0.60 [0.51-0.68], whereas the sensitivity and specificity of the method using PSGR, was of 0.59 [0.47-0.70] and the specificity of 0.73 [0.65-0.80].

Thus, in a preferred embodiment, the diagnostic method of the invention further comprises
(i) the determining the levels of PCA3 mRNA in a biofluid from said subject,
(ii) comparing the expression levels of said PSGR and PCA3 genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PSGR and PCA3 genes determined in a patient population being at risk of suffering prostate cancer,
wherein a significant increase in the expression level of at least one of said genes in said sample with respect to said predetermined cut off value for said gene is indicative that the subject suffers from PCa with said desired sensitivity.

As used herein, the term "PCA3" refers to the gene known as prostate cancer antigen 3 (Accesion number in NCBI is AF103907 or NR_015342) corresponds to a non-protein coding gene located on chromosome 9 which encodes a prostate-specific non-coding RNA which is highly over expressed in more than 95% of primary PCa specimens and PCa metastasis.

The term "cut off value", when referring to the expression levels PSGR and PCA3 genes, refer to a reference expression level indicative that a subject is likely to suffer prostate cancer with a given sensitivity if the expression levels of the patient are above said cut-off or reference leves. Typically, cut-off values are calculated using a Receiver Operating Characteristic curve (ROC curve).

In practice, Receiver Operating Characteristic curves (ROC curves), are typically calculated by plotting the value of a variable versus its relative frequency in "normal" (i.e. apparently healthy) and "disease" populations. For any particular marker or panel of markers (i.e., the expression levels of said PCA3 and PSGR genes), a distribution of marker levels for subjects with and without a disease will likely overlap. Under such conditions, a test does not absolutely distinguish normal from disease with 100 percent accuracy, and the area of overlap indicates where the test cannot distinguish normal from disease. A threshold or cut-off value is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art. See, e.g., Hartley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5 percent of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1- specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, particular thresholds for one or more markers in a panel are not relied upon to determine if a profile of marker levels obtained from a subject are indicative of a particular diagnosis/prognosis. Rather, the present invention may utilize an evaluation of a marker panel "profile" as a unitary whole. A particular "fingerprint" pattern of changes in such a panel of markers may, in effect, act as a specific diagnostic or prognostic indicator. As discussed herein, that pattern of changes may be obtained from a single sample, or from temporal changes in one or more members of the panel (or a panel response value). A panel herein refers to a set of markers.

As described herein after, a panel response value is preferably determined by plotting ROC curves for the sensitivity (i.e. true positives) of a particular panel of markers versus 1-(specificity) (i.e. false positives) for the panel at various cut-offs. In these methods, a profile of marker measurements from a subject is considered together to provide a global probability (expressed either as a numeric score or as a percentage risk) of a diagnosis or prognosis. In such embodiments, an increase in a certain subset of markers may be sufficient to indicate a particular diagnosis/prognosis in one patient, while an increase in a different subset of markers may be sufficient to indicate the same or a different diagnosis/prognosis in another patient. Weighting factors may also be applied to one or more markers in a panel, for example, when a marker is of particularly high utility in identifying a particular diagnosis/prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

For a marker measured on continuous scales, a ROC curve is a plot of true positive fraction versus false positive fraction, evaluated for all possible cut-off point values. For binary outcome, i.e., presence of prostate cancer or not, the ROC curve quantifies the discriminatory ability of a marker for separating cases from controls. The standard deviations of the area under the curve (AUC) and the differences between AUCs are computed with the U-statistic of DeLong et al. (Biometrics, 1988, 44:837-845), or the bootstrap re-sampling method.

Preferably, a ROC curve is determined for k markers by firstly using a k-component detection threshold for each biomarker followed by declaring the test as positive if at least one of the scores was above its detection threshold. Sensitivity and specificity values are tipically calculated over the range of k-component thresholds which allows the generation of a cloud of points. The optimal ROC curve points for the new marker can be obtained in the following way: for a fixed sensitivity value, the maximum value for specificity was selected among the range of specificities in the cloud of points that matched that sensitivity value.

In certain embodiments, markers and/or marker panels are selected to exhibit at least about 70 percent sensitivity, more preferably at least about 80 percent sensitivity, even more preferably at least about 85 percent sensitivity, still more preferably at least about 90 percent sensitivity, and most preferably at least about 95 percent sensitivity, combined with at least about 70 percent specificity, more preferably at least about 80 percent specificity, even more preferably at least about 85 percent specificity, still more preferably at least about 90 percent specificity, and most preferably at least about 95 percent specificity. In particularly preferred embodiments, both the sensitivity and specificity are at least about 75 percent, more preferably at least about 80 percent, even more preferably at least about 85 percent, still more preferably at least about 90 percent, and most preferably at least about 95 percent. The term "about" in this context refers to +/- 5 percent of a given measurement.

The skilled artisan will understand that associating a diagnostic or prognostic indicator, with a diagnosis or with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, e.g., Dowdy and Wearden, Statistics for Research, John Wiley and Sons, New York, 1983. Preferred confidence intervals of the invention are 90 percent, 95 percent, 97.5 percent, 98 percent, 99 percent, 99.5 percent, 99.9 percent and 99.99 percent, while preferred p values are 0.1, 0.05, 0.025, 0.02, 0.01, 0.005, 0.001, and 0.0001.

As used herein, "sensitivity" refers to the true positive fraction of disease subjects and it is calculated by TP/(TP+FN), wherein "TP" is true positive, which for a disease state test means correctly classifying a disease subject; and "FN" is false negative, which for a disease state test means classifying a disease subject incorrectly as non-disease or normal.

The term "specificity", as used herein, refers to the true negative fraction of non-disease or normal subjects, and it is calculated by TN/(TN+FP), wherein "TN" is true negative, which for a disease state test means classifying a non-disease or normal -subject correctly, and "FP" is false positive, which for a disease state test means classifying a normal subject incorrectly as having disease.

The expression "positive predictive value" or "PPV", as used herein, refers to the true positive fraction of all positive test results and it is calculated by TP/(TP+FP), wherein "TP" is true positive, which for a disease state test means correctly classifying a disease subject, and "FP" is false positive, which for a disease state test means classifying a normal subject incorrectly as having disease. It is inherently impacted by the prevalence of the disease and pre-test probability of the population intended to be tested.

The expression "negative predictive value" or "NPV", as used herein, refers to the true negative fraction of all negative test results, and it is calculated by TN/(TN + FN), wherein "TN" is true negative, which for a disease state test means classifying a non-disease or normal -subject correctly, and "FN" is false negative, which for a disease state test means classifying a disease subject incorrectly as non-disease or normal. It also is inherently impacted by the prevalence of the disease and pre-test probability of the population intended to be tested.

The term "patient population being at risk of suffering prostate cancer", as used herein, refers to a population of patients which have been diagnosed as showing risk of suffering prostate cancer based on one or more of the assays available for detecting prostate cancer. The population, for example, can comprise 3, 4, 5, 10, 15, 20, 30, 40, 50 or more subjects.

Patients being at risk of suffering prostate cancer include, without limitation, patients showing one or more of the following diagnostic traits:
- Positive for digital rectal examination (DRE), i.e. Presence of areas which are irregular, hard or lumpy detected by a procedure where the examiner inserts a gloved, lubricated finger into the rectum to check the size, shape, and texture of the prostate as described by Richie JP et al. (Urology, 1993, 42:365-74 and Carvalhal GF et al. (J. Urol., 1999, 161:835-9). Although the DRE only evaluates the back of the prostate, 85 percent of prostate cancers arise in this part of the prostate. Prostate cancer which can be felt on DRE is generally more advanced.
- Transrectal ultrasound, also known as prostate ultrasound, ultrasound scanning or sonography, involves exposing part of the body to high-frequency sound waves to produce pictures of the inside of the body. Because ultrasound images are captured in real-time, they can show the structure and movement of the body's internal organs, as well as blood flowing through blood vessels.
- Elevated PSA levels in blood: Although no standard exists as to the decision level for biopsies in patients showing increased PSA levels in blood, different values of PSA have been chose arbitrarily as decision level, in particular, levels above 4 ng/ml were chosen as decision level for biopsies in the clinical trial upon which the FDA in 1994 based adding prostate cancer detection in men age 50; 4 ng/mL was used as the biopsy decision level in the PLCO trial, 3 ng/mL was used in the ERSPC and ProtecT trials, and 2.5 ng/mL is used in the 2007 NCCN guideline. PSA levels can change for many reasons other than cancer. Two common causes of high PSA levels are enlargement of the prostate (benign prostatic hypertrophy (BPH)) and infection in the prostate (prostatitis).
- Increased levels in blood of the prostatic acid phosphatase (PAP).

In a method of the invention, the cut-off values for the expression of PSGR and PCA3 genes can be selected such that, when the sensitivity is at least about 70 percent, the specificity of diagnosing an individual is in the range of 70-100 percent, for example, at least 75 percent, 80 percent, 85 percent, 90 percent or 95 percent in at least 60 percent of the patient population assayed, or in at least 65 percent, 70 percent, 75 percent or 80 percent of the patient population assayed.

In a preferred embodiment, the patients of the patient population being at risk of suffering prostate cancer are patients having PSA levels above 4 ng/ml and/or patients with a positive DRE. In a still more preferred embodiment, the patient population being at risk of suffering prostate cancer is formed by patients having PSA levels found within the so-called grey zone which corresponds to PSA levels in serum or blood associated with an undesirably large numbers of false positives and false negatives. Typically, the grey zone corresponds to PSA levels higher than 4 ng/ml and lower than 10 ng/ml.

In a more preferred embodiment, the subject wherein the method of the invention is carried out is a patient wherein no previous biopsy has been carried out.

Determination of the PCA3 expression levels is carried essentially as explained above for PSGR. Moreover, although the determination of PSGR and PCA3 expression levels can be carried out in different biofluids, it is preferred that both genes are determined in the same biofluid. In a preferred embodiment, the biofluid is urine. In a still more preferred embodiment, the biofluid is urine obtained after a prostate massage.

In a preferred embodiment, the expression levels of the PSGR and PCA3 mRNA levels are normalized using the expression levels of a housekeeping gene to take into consideration that an increased expression levels of the genes in the sample may not be the result of the malignant proliferation of the prostate cells but rather of a non-malignant prostate growth as it occurs for instance in the benign hyperplasia of the prostate. Moreover, the method can be carried out in a sample containing cells from tissues other than prostate which express these markers. In particular, if the method is carried out in urine sediments, besides cancer cells, it is possible to find cells from the urothelium, kidney, bladder or blood. Thus, in a preferred embodiment, the expression levels of the different genes forming the marker panel are normalized to the amount of a housekeeping gene.

The term "normalization", as used herein, means conversion of the quantitative numerical values into numerical values which can be compared with gene expression amounts obtained by other gene expression analyses. Typically, normalization is carred out by using the gene expression levels of a housekeeping gene being steadily expressed is used as an index for normalization of gene expression amounts.

The term "housekeeping gene", as used herein, refers to a gene which is generally ubiquitously expressed in all tissues. These genes encode proteins that provide the basic, essential functions that all cells need to survive. Housekeeping genes are usually expressed at the same level in all cells and tissues, but with some variances, especially during cell growth and organism development. Commonly used housekeeping genes include, without limitation, GAPDH (glyceraldehyde-3-phosphate dehydrogenase), beta-actin gene, the tubulin gene, genes coding for the 18S or 28S rRNA subunits of the ribosome. An exemplary listing of housekeeping genes can be found, for example, in Trends in Genetics, 19, 362-365 (2003).

In a preferred embodiment, the housekeeping gene is a "prostate housekeeping gene". As used herein, the term "prostate housekeeping gene" refers to a gene which is expressed at steady levels in prostate cells irrespective of whether the cells are normal prostate cells or prostate adenocarcinome cells. This gene allows the detection of the number of prostate derived cells in a sample

Suitable prostate housekeeping genes for use in the present invention include, without limitation, PSA (Sokoll et al., 1997, Urol. Clin. North Am. 24:253-259), DD3 (Cancer Res., 1999, 59:5975-9), HPG-1, PSM, NKX3, six-transmembrane epithelial antigen of prostate (STEAP), Trp-p8 (Tsvaler et al.), prostatic acid phosphatase, creatine kinase, thymosin b-15, HPC1 basic prostate gene, the prostate-specific glandular kallikrein protein hK2 encoded by the hKLK2 gen, the prostate-specific antigen protein, hK3 (PSA) encoded by the hKLK3 gene, the SIM2 gene (WO09135019A), the ERG gene (WO09135019A), TMPRSS2, PSM or prostate-specific membrane antigen (Fair et al., 1997, Prostate 32:140-148), PSCA or prostate stem cell antigen (Reiter et al., 1998. Proc. Natl. Acad. Sci. USA 95:1735-1740), TMPRSS2 (Lin et al., 1999. Cancer Res. 59:4180-4184), PDEF (Oettgen et al., 2000, J. Biol. Chem. 275:1216-1225), PSG-1 or prostate-specific gene-1 (Herness, 2003, Cancer Res. 63:329-336). PCA3 (Bussemakers et al., 1999. ,Cancer Res. 59:5975-5979], WO98/045420, WO01/0235 WO2004/070056, WO2005/003387), PCGEM1 (Srikantan et al., 2000. Proc. Natl. Acad. Sci. USA 97:12216-12221) and the genes P704P, P712P, and P775P (Stolk et al., 2004. Prostate 60:214-226).

In a preferred embodiment, the prostate housekeeping gene is the PSA gene. The term PSA, as used herein, refers to a gene located on chromosome 19 which encodes a glycoprotein with serine protease activity expressed under androgen control by glandular epithelial cells of the prostate and secreted into seminal plasma to liquefy it. PSA protein is normally confined to the prostate but in the case of prostatic disease such as cancer or BPH (benign prostate hyperplasia), PSA leaks into the blood where it is present in different forms, including one that is and one that is not bound to protein complexes (El-Shirbiny, 1994, Adv. Clin. Chem. 31:99). The measurement of total PSA serum concentrations is one of the most frequently used and FDA approved biochemical tests in the screening and management of prostate cancer patients. Studies to date have suggested that screening with PSA, in conjunction with digital rectal exams and transrectal ultrasound, increases the detection of early prostate cancers often while still localized to the gland itself (Brawer et al., 1992, J. Urol. 147:841). Serum PSA is also useful for monitoring of patients after therapy, especially after surgical prostatectomy. However, total PSA measurements also identify a large number of patients with abnormally elevated levels who are subsequently found to have no prostate cancer. Recently, the concept of measuring the percentage free/total PSA ratio was shown to increase the specificity of prostate cancer screening in men with PSA between 4 and 10 ng/ml (Letran et al., 1998, J. Urol. 160:426).

### Method for assessing or monitoring the response to a therapy in a subject having prostate cancer

The teachings of the invention can be used for monitoring and determining the efficacy of the therapy administered to a subject having PCa.

Thus, in another aspect, the invention relates to a method for assessing or monitoring the response to a therapy in a subject having prostate cancer (PCa) which comprises comparing
(i) determining the expression levels of the PSGR mRNA in an biofluid sample from said subject after administering said therapy,
(ii) comparing the levels obtained in step (i) with reference value for said mRNA obtained prior to administration of the therapy
wherein a significant decrease in the PSGR mRNA levels for said gene in the biofluid after the administration of said therapy in comparison with the level of expression of each of said genes prior to the administration of the therapy in the subject sample is indicative that the therapy administered to the subject is efficacious.

Alternatively, the invention relates to a method for assessing or monitoring the response to a therapy in a subject having prostate cancer (PCa) which comprises
(i) determining the expression levels of the PSGR mRNA in an biofluid sample from said subject after administering said therapy,
(ii) comparing the levels obtained in step (i) with reference value for said mRNA obtained prior to administration of the therapy
wherein a significant increase or lack of change in the PSGR mRNA levels for said gene in the biofluid after the administration of said therapy in comparison with the level of expression of each of said genes prior to the administration of the therapy in the subject sample is indicative that the therapy administered to the subject is inefficacious.

As used in the present invention, the expression "assessing or monitoring the response to therapy" relates to the possibility of determining the response of a subject having PCa to the therapy administered to said subject. In PCa therapy, a variety of treatments can be used in an attempt to eliminate or contain the cancer. Treatment for PCa may involve active surveillance, hormonal therapy, surgery (e.g., radical prostatectomy, transurethral resection of the prostate (TURP), cryosurgery), radiation therapy including brachytherapy (prostate brachytherapy) and external beam radiation, High Intensity Focused Ultrasound (HIFU), chemotherapy, or some combination. Which option is best depends on the stage of the disease, the Gleason score, and the PSA level. Other important factors are the man's age, his general health, and his feelings about potential treatments and their possible side effects. Because all treatments can have significant side effects, such as erectile dysfunction and urinary incontinence, treatment discussions often focus on balancing the goals of therapy with the risks of lifestyle alterations.

Briefly, depending on the above mentioned factors (e.g., age of the subject, as well as the size, location and cancer phase), (i) surgery, consisting of the surgical extirpation of the tumor (ii) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and antitumor agents; and/or (iii) immunotherapy, wherein the administered compound stimulates, enhances or strengthens the natural function of the immune system against cancer to recognize and eliminate the cancerous cells from the body, can be used. Thus, the method of the invention allows determining the response of the subject having PCa to any treatment, particularly, to any cytotoxic and/or cytostatic treatment and, more specifically, to a treatment by means of chemotherapy, radiotherapy, antitumor agents or combinations thereof.

Suitable chemotherapy agents include but are not limited to alkylating agents [e.g., Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, Uramustine, etc.]; anti-metabolites [e.g., purine (azathioprine, mercaptopurine), pyrimidine (Capecitabine, Cytarabine, Fluorouracil, Gemcitabine), folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., Gleevec^{(™)}] ; an epidermal growth factor receptor inhibitor [e.g., Iressa^{(™)}, erlotinib (Tarceva^{(™)}), gefitinib, etc.]; an FPTase inhibitor [e.g., FTIs (Rl 15777, SCH66336, L- 778,123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltirexed (ZD 1694, Tomudex), ZD9331, 5-FU, etc.]; an S-adenosyl-methionine decarboxylase inhibitor [e.g., SAM468A, etc.]; a DNA methylating agent [e.g., TMZ, etc.]; a DNA binding agent [e.g., PZA, etc.]; an agent which binds and inactivates O⁶-alkylguanine AGT [e.g., BG]; a c-ra/-1 antisense oligo-deoxynucleotide [e.g., ISIS-5132 (CGP- 69846A)]; tumor immunotherapy; a steroidal and/or non-steroidal antiinflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Suitable chemotherapy agents are described with more detail in the literature, such as in The Merck Index in CD-ROM, 13th edition.

According to this inventive aspect, the expression level of PSGR determined in the sample from a subject having PCa obtained at a first period of time (first subject sample) and the expression level of PSGR determined in the sample from a subject having PCa obtained at a second period of time (second subject sample) are compared allowing the efficacy of the response to therapy in said subject having PCa to be assessed or monitorized. The second subject sample can be taken from the same subject having PCa from which the first measure is derived, at a second period of time, i.e., at any time after the first period of time, e.g., one day, one week, one month, two months, three months, 1 year, 2 years, or more after the first subject sample. In a particular embodiment, the first subject sample is taken prior to the subject receiving treatment, e.g. chemotherapy, radiation therapy, or surgery, and the second subject sample is taken after treatment. In another particular embodiment, the first subject sample is taken after the subject has started/received treatment, e.g. chemotherapy, radiation therapy, or surgery, and the second subject sample is taken later, at different time periods during a course of treatment which efficacy is to be assessed or monitorized. These methods allow for the evaluation of a particular treatment for a selected subject previously diagnosed with PCa. Consequently, if the therapy is not efficacious for treating PCa in said subject, then said therapy should be changed and a new therapy should be designed to treat PCa in said subject. The course of the new treatment can be easily followed according to this method.

As mentioned previously concerning the diagnostic method of the invention, the level of expression of the biomarker gene (PSGR) can be determined by any suitable means known in the art, such as, for example, qPCR. The measurement is obtained under conditions that are substantially repeatable.

Once the levels of the PSGR mRNA in the subject samples, at different periods of time (first and second subject samples) have been determined, it is necessary to identify if there is a significant decrease in the expression of each one of said genes in the second subject sample in comparison with the expression levels of said gene biomarker in the first subject sample. A decrease in the expression of a gene in the second subject sample under study is considered a "significant decrease" with respect to the expression level of said biomarker genes in said first subject sample when the expression level in the second subject sample decreases with respect to the expression level thereof in the first subject sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Similarly, an increase in the expression of a gene in the second subject sample under study is considered a "significant increase" with respect to the expression level of said biomarker gene in said first subject sample when the expression level in the second subject sample increases with respect to the expression level thereof in the first subject sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

Similarly, a lack of change in the expression of a gene in the second subject sample under study with respect to the expression level of said biomarkers genes in said first subject sample when the expression level in the second subject sample indicates that the expression levels are substantially constant between the two measurements. By way of example, constant expression levels indicate that the first measurement is not more than 105%, not more than 104%, not more than 103%, not more than 102%, not more than 101%, not less than 99%, not less than 98%, not less than 97%, not less than 97%, not less than 96% or not less than 95%.

Thus, a significant decrease in the mRNA levels of PSGRin the second subject sample with respect to the expression level of said biomarker gene in the first subject sample is indicative that the therapy administered to the subject having PCa is efficacious. On the contrary, if no significant decrease in the level of expression of biomarker gene in the second subject sample with respect to the expression level of said biomarker gene in the first subject sample is achieved, or even, if a significant increase in the level of expression of said biomarker gene in the second subject sample with respect to the expression level of each of said biomarker gene in the first subject sample is achieved, then the therapy administered to said subject is not efficacious for treating PCa in said subject; consequently, said therapy should be changed and a new therapy should be designed to treat PCa in said subject. The course of the new treatment can be easily followed according to this method.

Moreover, the method for assessing or monitoring the response to PCa therapy according to the present invention can be further improved by combining the mRNA levels of the PSGR gene with the mRNA levels of a second marker, namey, PCA3. Thus, a that a therapy is considered as being efficacious when the mRNA level of at PSGR or PCA3 is lower than the cut-off value. Thus, in another aspect, the method for monitoring the response to a therapy according to the present invention is carried out by performing steps (i) and (ii) as defined above but wherein steps (i) and (ii) further comprise the determination of the PSGR mRNA levels in a biofluid from the patient, wherein step (iii) further comprises comparing the PCA3 level in the patient with a predetermined cut-off value for PCA3 wherein said predetermined cut off value corresponds to a PCA3 value which correlates with the highest specificity at said desired sensitivity in a ROC (receiver operating characteristics) curves calculated based on the expression levels of the PSGR and PCA3 determined in a patient population being at risk of suffering prostate cancer. Finally, the method allows obtaining a conclusion as to whether a therapy is being efficacious if an increased expression level of at least one of said genes in said sample with respect to said predetermined cut off value for said gene or increased PSAD values with respect to said predetermined cut off value is detected or whether a therapy is being inefficacious wherein a significant increase or a lack of change in the expression level of at least one of said genes in the biofluid after the administration of said therapy in comparison with the level of expression of each of said genes prior to the administration of the therapy in the subject sample.

### Monitoring the progression of PCa in a subject

The teachings of the invention can also be used for monitoring the progression of PCa in a subject. Thus, in another aspect, the invention relates to a method for monitoring the progression of prostate cancer (PCa) in a subject comprising
(i) determining the expression levels of the PSGR mRNA in an urine sample from said subject,
(ii) comparing the levels obtained in step (i) with reference value for said mRNA obtained from the same subject at an earlier time point of the disease wherein a significant decrease in the PSGR mRNA for said gene in the biofluid in comparison with the level of expression of said mRNA at the earlier time point is indicative that the prostate cancer shows a good progression or
wherein a significant increase or a lack of change in the PSGR mRNA for said gene in the biofluid in comparison with the level of expression of said mRNA at the earlier time point is indicative that the prostate cancer shows a bad progression.

As used in the present invention, the expression "monitoring the progression of PCa", which is equivalent to "determining the prognosis", relates to the determination of one or several parameters indicating the progression of the disease in a patient diagnosed with PCa. Parameters suitable for determining the evolution of a subject diagnosed with PCa are selected from the group of tumor response, risk of relapse, disease-free survival and/or overall survival of the subject. As used herein, the expression "risk of relapse" is understood as the probability of a subject developing PCa and/or a secondary metastasis again after a disease-free period; "disease-free survival" is understood as the time period after the treatment in which the cancer is not found; and "overall survival of the subject" is understood as the percentage of subjects who survive, from the time of the diagnosis or treatment, after a defined time period.

According to this inventive aspect, the mRNA level of PSGR determined in the sample from a subject having PCa obtained at a first period of time (first subject sample) and the mRNA level of PSGR determined in the sample from a subject having PCa obtained at a second period of time (second subject sample) are compared allowing the progression of PCa in said subject having PCa to be monitorized. The second subject sample can be taken from the same subject having PCa from which the first measure is derived, at a second period of time, i.e., at any time after the first period of time, e.g., one day, one week, one month, two months, three months, 1 year, 2 years, or more after the first subject sample. In a particular embodiment, the first subject sample is taken prior to the subject receiving treatment, e.g. chemotherapy, radiation therapy, or surgery, and the second subject sample is taken after treatment. In another particular embodiment, the first subject sample is taken after the subject has started/received treatment, e.g. chemotherapy, radiation therapy, or surgery, and the second subject sample is taken later, at different time periods during a course of treatment. These methods allow for the evaluation of the progression of the PCa in a selected subject previously diagnosed as suffering from PCa. Consequently, if the PCa has a bad prognosis, a further therapy should be designed to treat PCa in said subject. The progression of the PCa after said new treatment can be easily followed according to the teachings of this invention.

As mentioned previously concerning the diagnostic method of the invention, the mRNA levels of the biomarker gene (PSGR) can be determined by any suitable means known in the art, such as, for example, qPCR. The measurement is obtained under conditions that are substantially repeatable.

Once the expression levels of the biomarker gene (PSGR) in the subject samples, at different periods of time (first and second subject samples) have been determined, it is necessary to identify if there is a significant increase in the expression of each one of said genes in the second subject sample in comparison with the expression levels of said gene biomarker in the first subject sample. Alternatively, if desired, one may analyze if there is a significant decrease in the expression of each one of said genes in the second subject sample in comparison with the expression levels of said gene biomarker in the first subject sample. The terms "significant increase" and "significant decrease" applied to the expression levels of biomarker gene have been previously defined.

Thus, a significant increase in the mRNA level of each of said biomarker gene in the second subject sample with respect to the expression level of each of said biomarker gene in the first subject sample is indicative that PCa in the subject under study is in progression (i.e., it has a bad prognosis); thus, the therapy administered to the subject under study should be changed and a new therapy should be designed to treat PCa in said subject. The progression of the PCa in the subject can be easily followed according to this method.

On the contrary, if no significant increase in the level of mRNA of said biomarker gene in the second subject sample with respect to the expression level of each of said biomarker genes in the first subject sample is achieved, or even, if a significant decrease in the level of expression of each of said biomarker genes in the second subject sample with respect to the expression level of each of said biomarker genes in the first subject sample is achieved, then PCa in the subject under study is not in progression (i.e., it does not have a bad prognosis).

Moreover, the method for monitoring the progression of a prostate cancer patient according to the present invention can be further improved by combining the mRNA levels of the PSGR gene with the mRNA levels of a second marker, namely, PCA3. Thus, a significant increase in the mRNA level of at least one said biomarker genes in the second subject sample with respect to the expression level of each of said biomarker gene in the first subject sample is indicative that PCa in the subject under study is in progression (i.e., it has a bad prognosis)

On the contrary, if no significant increase in the level of mRNA of at least one of said biomarker genes in the second subject sample with respect to the expression level of each of said biomarker genes in the first subject sample is achieved, or even, if a significant decrease in the level of expression of each of said biomarker genes in the second subject sample with respect to the expression level of each of said biomarker genes in the first subject sample is achieved, then PCa in the subject under study is not in progression (i.e., it does not have a bad prognosis).

The progression of the PCa in the subject can be easily followed according to this method.

The invention is detailed below by means of the following examples which are merely illustrative and by no means limiting for the scope of the invention.

### EXAMPLES

### MATERIAL AND METHODS

### Patients and urine collection

This study was approved by the institutional review board of the Vall d'Hebron Hospital. All urine samples were obtained from the Department of Urology of the Vall d'Hebron Hospital in Barcelona and were taken from patients subjected to prostate biopsy because of their increased serum PSA levels (>4.0 ng/ml) and/or an abnormal diagnostic DRE. Patients with other known tumors and/or previous PCa therapies were excluded from the study. Written informed consent was obtained from all patients. 262 urine sediments were collected immediately after prostate massage (PM). Prostate massages were performed by systematically applying severe pressure to the prostate from the base to apex and from the lateral to the median line of each lobe. The definitive diagnosis of all patients was achieved by prostate biopsy. Biopsies were performed using an end-fire ultrasound transducer (Falcon 2101, B-K Medical, Inc.) and an automatic 18 gauge needle (Bard, Inc.). The minimum number of cores removed in every procedure was 10, and between 1 and 8 additional cores were removed, according to the Vienna nomogram. The study population consisted of 215 men, 73(34%) were positive for PCa and 142 (66%) were negative (benign controls without cancer) (Table 1). The clinical and pathological information for these 215 patients are shown in Table 1.

**Table 1. Clinical and Pathological Information of the patients used in this study**

| | | All patients |
|---|---|---|
| Clinical information | | average (min, max) |
| No of patients | | 215 |
| Age (yr) | | 65.7 (44 - 85) |
| PSA level (ng/ml) | | 11.86 (1.5 - 189) |
| Prostate Volume (ml) | | 48.52 (10.0 - 162.0) |
| PSA density (PSAD) | | 0.3 (0.04 - 5.37) |
| Ratio free PSA / total PSA | | 0.17 (0.00 - 0.26) |
| Positive DRE | | Controls 30/142 (42.6%) |
| | | Cancers 27/73 (19.7%) |
| Prostate cancer (prevalence) | | 73 (34%) |
| | GLEASON < 7 | 18 (24.7%) |
| | GLEASON = 7 | 42 (57.5%) |
| | GLEASON > 7 | 13 (17.8%) |
| Controls without cancer (prevalence) | | 142 (66%) |

### Sample preparation

Urine samples (30-50 ml first catch) were collected after PM. Urine was collected in urine collection cups, kept on ice, transported to the lab and processed within 30 min. The urine samples were centrifuged at 2500g for 10 minutes at 4°C, and then the pellets were washed twice with cold PBS 1X. Finally, the pellets were stored with 1:5 RNA Later (Ambion) at 80°C until RNA extraction.

### RNA isolation and preamplification

Urine RNA was extracted with the QIAamp ® Viral RNA Mini Kit (Qiagen). Single-stranded cDNA synthesis was carried out using the SuperScript III Reverse Transcriptase (Invitrogen) and incubated at 37°C for 20 min with RNaseH (Invitrogen). cDNA was stored at -20°C until preamplification with the TaqMan Preamp Master Mix Kit (Applied Biosystems).

### Quantitative PCR Analysis

Quantitative real time PCR (qPCR) was used to detect the prostate derived transcripts PSGR, PCA3 and the control transcript PSA, all using the TaqMan ® Gene Expression Assay (Applied Biosystems). Reactions were carried out in triplicate on an ABI-Prism-7900 qPCR machine, and only those results with a standard deviation value of <0.38 were accepted (as recommended by the manufacturer). Threshold levels were set into the exponential phase of the qPCR. The data analysis was carried out using the ABI-Prism-7900SDS Software V2.3 (Applied Biosystems, Foster City, CA) with the same baseline and threshold set for each plate to generate threshold cycle (Ct) values for all the genes in each sample. We used PSA as a prostate housekeeping gene for normalization (Hessels D. et al. 2003, Eur. Urol. 44:8-15). We established a qPCR cut-off point of ct (PSA) < 30 after preamplification to decide whether a specimen was informative and the amount of RNA present sufficient to yield an accurate result. We selected this cut-off point since higher ct values would correspond to approximately less than 10 prostatic cells per reaction. For each marker a score was calculated as ct (marker) / ct (PSA) × 1000 (Hessels D. et al., 2009, Nat. Rev. Urol., 6:255-261; Marks LS. et al., 2007, Urology, 69:532-535 and van Gils M.P. et al., 2007, Clin Cancer Res. 13:939-943). A cut-off point for each marker was determined, and a specimen was counted positive when the marker was over the cut-off point.

### Statistical Analysis

In the cases of PCa and in the negative biopsy individuals, the characterization of candidate biomarkers was accomplished by comparing their mean values (univariate analysis). The T-test with Welch correction was used when the distribution of the data was normal. The T-test was carried out on log-transformed data, which were applied to stabilize the variances. ANOVA analysis was used to compare more than 2 groups of variables. Receiver Operating Characteristic (ROC) curves and the Area under the Curve (AUC) were used to evaluate the performance of each marker score as a measure to discriminate between patients in the PCa group and the others. ROC curves were calculated individually for each marker and for combinations of both markers, as well as for PSA, Multivariate ROC (MultiROC). In order to combine more than one marker (*k*), the following procedure was followed: first, a k-component detection threshold was used, one for each biomarker; then, the new marker was declared positive if at least one of the scores was above its detection threshold. Sensitivity and specificity values were calculated over the range of the k-component thresholds. This generated a cloud of points. Optimal ROC curve points for the new marker were obtained in the following way: for a fixed sensitivity value, the maximum value for specificity was selected from among the range of specificities in the cloud of points that matched that sensitivity value. The Area under the MultiROC curve (AUCm) was measured to compare the outcome prediction performance between the paired combination and the individual markers. In order to determine if the combined model could predict the outcome significantly better than the individual biomarkers at a fixed sensitivity, we compared the corresponding false positive rates using a z test for proportions. The computation of the variances of the false positive rates for the z test was carried out as explained in Krzanowski et al. for PSGR and PCA3 alone. We used a resampling approach in the case of the combination of PSGR and PCA3 (PSGR +PCA3). P-values in these comparisons were corrected for multiple testing problems following the false discovery rate procedure. Statistical analyses were performed using the PASW V17.0, Graphpad V4.0 and R.

### RESULTS

For the subject group studied, 215 of the 262 initial specimens yielded sufficient prostate derived RNA for analysis, corresponding to an informative specimen rate of 82.1% (benign 76.7% and PCa 23.3%).

PCA3 and PSGR were tested by univariate analysis to see whether they could differentiate between the patients with PCa and the patients with negative needle biopsies. We found that PSGR (p=0.008) and PCA3 (p<0.001) were significant predictors for PCa (Figure 1).

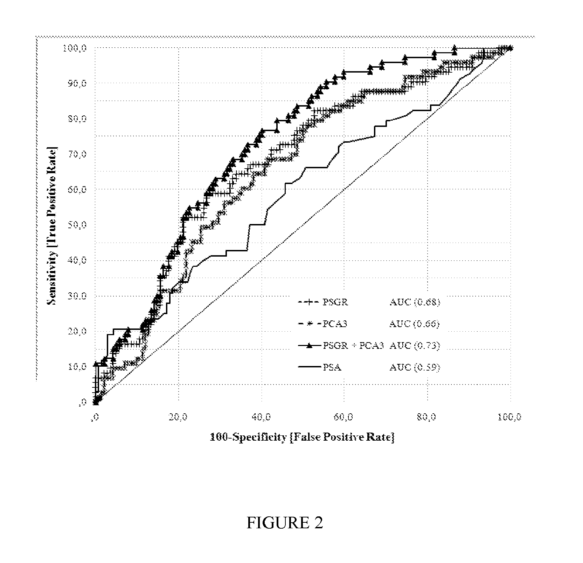

To visualize the diagnostic efficacy and to summarize the data of the gene-based qPCR assay of urine samples, a ROC curve analysis (table 2) was performed for each marker. The ROC curve was used to determine the sensitivity and specificity at different score cut-offs (Figure 2).

**Table 2. Optimal cut-off value of PCA3 and PSRG combination**

| Cut-off | | TP/(TP+FN) Sensitivity | TN/(TN+FP) Specificity |
|---|---|---|---|
| PCA3 | PSGR | | |
| 0,18 | 12,71 | 1,00 | 0,13 |
| 0,52 | 13,50 | 0,99 | 0,18 |
| 1,02 | 13,50 | 0,97 | 0,25 |
| 1,02 | 24,40 | 0,96 | 0,31 |
| 1,66 | 13,50 | 0,95 | 0,34 |
| 1,56 | 24,40 | 0,93 | 0,40 |
| 1,56 | 38,41 | 0,92 | 0,42 |
| 1,66 | 38,41 | 0,90 | 0,44 |
| 1,74 | 38,41 | 0,89 | 0,46 |
| 1,66 | 59,90 | 0,88 | 0,46 |
| 1,74 | 59,90 | 0,86 | 0,48 |
| 1,81 | 59,90 | 0,85 | 0,49 |
| 5,27 | 24,40 | 0,84 | 0,51 |
| 5,50 | 24,40 | 0,82 | 0,52 |
| 5,27 | 33,45 | 0,81 | 0,54 |
| 7,23 | 24,40 | 0,79 | 0,56 |
| 5,71 | 33,45 | 0,78 | 0,56 |
| 7,23 | 33,45 | 0,77 | 0,60 |
| 7,23 | 38,41 | 0,75 | 0,61 |
| 7,23 | 43,77 | 0,74 | 0,61 |
| 7,23 | 50,96 | 0,73 | 0,63 |
| 7,23 | 59,90 | 0,71 | 0,64 |
| 7,23 | 79,07 | 0,70 | 0,65 |
| 41,82 | 24,40 | 0,68 | 0,67 |
| 52,09 | 24,40 | 0,67 | 0,68 |
| 53,96 | 24,40 | 0,66 | 0,68 |
| 15,48 | 50,96 | 0,64 | 0,69 |
| 41,82 | 33,45 | 0,63 | 0,71 |
| 52,09 | 33,45 | 0,62 | 0,72 |
| 53,96 | 33,45 | 0,60 | 0,73 |
| 81,42 | 33,45 | 0,59 | 0,73 |
| 394,13 | 26,87 | 0,58 | 0,73 |
| 126,47 | 33,45 | 0,56 | 0,75 |
| 202,70 | 33,45 | 0,55 | 0,77 |
| 394,13 | 33,45 | 0,53 | 0,78 |
| 3725,93 | 33,45 | 0,52 | 0,79 |
| 15,48 | 147,35 | 0,51 | 0,75 |
| 53,96 | 50,96 | 0,49 | 0,77 |
| 202,70 | 38,41 | 0,48 | 0,79 |
| 394,13 | 38,41 | 0,47 | 0,80 |
| 3725,93 | 38,41 | 0,45 | 0,80 |
| 202,70 | 43,77 | 0,44 | 0,80 |
| 394,13 | 43,77 | 0,42 | 0,82 |
| 202,70 | 50,96 | 0,41 | 0,82 |
| 41,82 | 112,24 | 0,40 | 0,78 |
| 394,13 | 50,96 | 0,38 | 0,84 |
| 3725,93 | 46,26 | 0,37 | 0,83 |
| 3725,93 | 50,96 | 0,36 | 0,85 |
| 202,70 | 59,90 | 0,34 | 0,83 |
| 202,70 | 65,51 | 0,33 | 0,84 |
| 202,70 | 79,07 | 0,32 | 0,85 |
| 394,13 | 65,51 | 0,30 | 0,85 |
| 394,13 | 79,07 | 0,29 | 0,86 |
| 3725,93 | 65,51 | 0,27 | 0,86 |
| 394,13 | 87,29 | 0,26 | 0,87 |
| 394,13 | 99,94 | 0,23 | 0,88 |
| 394,13 | 112,24 | 0,22 | 0,89 |
| 394,13 | 147,35 | 0,21 | 0,92 |
| 394,13 | 179,10 | 0,19 | 0,93 |
| 3725,93 | 147,35 | 0,18 | 0,94 |
| 3725,93 | 179,10 | 0,16 | 0,95 |
| 3725,93 | 193,63 | 0,15 | 0,96 |
| 394,13 | 282,55 | 0,14 | 0,96 |
| 394,13 | 459,70 | 0,12 | 0,98 |
| 3725,93 | 240,97 | 0,11 | 0,96 |
| 772,58 | 459,70 | 0,10 | 0,98 |
| 3725,93 | 459,70 | 0,08 | 1,00 |

ROC curves (PSGR, PCA3 and PSA) and MultiROC (PSGRvPCA3) analysis were used to assess the outcome predictive values of the individual and the paired biomarkers. The following AUC values were obtained: PSGR: 0.68 [0.58-0.73], PCA3: 0.66 [0.61-0.76], PSA: 0.602 [0.52-0.68] and PSGRvPCA3: 0.73 [0.65-0.80]. Comparisons were made between them with the following results: (PSGRvPCA3) vs. PSGR p=0.124, (PSGRvPCA3) vs. PCA3 p=0.049 and PCA3 vs. PSGR p=0.663.

By maximizing the sum of sensitivity and specificity for PSGR, the assay sensitivity was 0.59 [0.47-0.70] and the specificity 0.73 [0.65-0.80]. For PCA3 the sensitivity was 0.69 [0.56-0.79] and the specificity 0.59 [0.50-0.67]. For the combined marker model (PSGRvPCA3), we achieved a sensitivity of 0.77 [0.65-0.85] and a specificity of 0.60 [0.51-0.68]. The positive predictive value for the combined model was (+PV) = 0.50 and the negative predictive value (-PV) = 0.83.

When the PSGR, PCA3 and PSGRvPCA3 were compared by fixing the sensitivity to the clinically interesting value of 95% [0.86-0.98], specificities of 0.15 [0.10-0.22] (PSGR), 0.17 [0.11-0.24] (PCA3) and 0.34 [0.26-0.42] (PCA3+PSGR) were obtained. Finally, a standard z test, in a range of sensitivities (90% to 97.5%), was performed to check if these differences were significant. For the sensitivity of 95%, the following p-value were obtained: PSGRvPCA3 compared to PSGR; p=0.049, PSGRvPCA3 compared to PCA; p=0.044 and PSGR compared to PCA3; p=1 (table 3).

**Table 3. Comparison of PSGR and PCA3 by z test for proportions**

| | Specificity | | | p value | | |
|---|---|---|---|---|---|---|
| | | | | PSGR vs. | PCA3 vs. | PSGR vs. |
| Sensitivity | PSGR | PCA3 | PSGRvPCA3 | 2M* | 2M* | PCA3 |
| 0.975 | 0.092 | 0.092 | 0.254 | 0.049 | 0.044 | 1 |
| 0.945 | 0.148 | 0.169 | 0.338 | 0.049 | 0.044 | 1 |
| 0.904 | 0.239 | 0.254 | 0.444 | 0.043 | 0.044 | 1 |

| | | | | | | |
|---|---|---|---|---|---|---|
| *2M: PSGR in combination with PCA3 (PSGRvPCA3) | | | | | | |

## Claims

1. A method for the diagnosis of prostate cancer in a subject comprising
(i) determining the levels of PSGR mRNA in a biofluid from said subject and
(ii) comparing the levels obtained in step (i) with reference values of said mRNA
wherein increased levels of PSGR mRNA with respect to the reference value is indicative that the subject suffers prostate cancer.

2. A method according to claim 1 further comprising
(i) determining the levels of PCA3 mRNA in a biofluid from said subject
(ii) comparing the expression levels of said PSGR and PCA3 genes with predetermined cut off values for each of said genes wherein said predetermined cut off values for each gene correspond to the expression level of said gene which correlates with the highest specificity at a desired sensitivity in a ROC (receiver operating characteristics) curve calculated based on the expression levels of the PSGR and PCA3 genes determined in a patient population being at risk of suffering prostate cancer,
wherein a significant increase in the expression level of at least one of said genes in said sample with respect to said predetermined cut off value for said gene is indicative that the subject suffers from PCa with said desired sensitivity.

3. A method for assessing or monitoring the response to a therapy in a subject having prostate cancer (PCa) which comprises
(i) determining the expression levels of the PSGR mRNA in an biofluid sample from said subject after administering said therapy,
(ii) comparing the levels obtained in step (i) with reference value for said mRNA obtained prior to administration of the therapy
wherein a significant decrease in the PSGR mRNA levels for said gene in the biofluid after the administration of said therapy in comparison with the level of expression of each of said genes prior to the administration of the therapy in the subject sample is indicative that the therapy administered to the subject is efficacious
or wherein a significant increase or lack of change in the PSGR mRNA levels for said gene in the biofluid after the administration of said therapy in comparison with the level of expression of each of said genes prior to the administration of the therapy in the subject sample is indicative that the therapy administered to the subject is inefficacious.

4. A method according to claim 3 further comprising
(i) determining the levels of PCA3 mRNA in a biofluid from said subject after the administering said therapy,
(ii) comparing the expression levels of said PSGR and PCA3 genes obtained before and after the administering of said therapy with predetermined cut off values for each of said genes wherein said predetermined cut off values for each gene correspond to the expression level of said gene which correlates with the highest specificity at a desired sensitivity in a ROC (receiver operating characteristics) curve calculated based on the expression levels of the PSGR and PCA3 genes determined in a patient population being at risk of suffering prostate cancer,
wherein a significant decrease in the expression level of at least one of said genes in the biofluid after the administration of said therapy in comparison with the level of expression of each of said genes prior to the administration of the therapy in the subject sample is indicative that the therapy administered to the subject is efficacious
wherein a significant increase or a lack of change in the expression level of at least one of said genes in the biofluid after the administration of said therapy in comparison with the level of expression of each of said genes prior to the administration of the therapy in the subject sample is indicative that the therapy administered to the subject is inefficacious.

5. A method for monitoring the progression of prostate cancer (PCa) in a subject comprising
(i) determining the expression levels of the PSGR mRNA in an urine sample from said subject,
(ii) comparing the levels obtained in step (i) with reference value for said mRNA obtained from the same subject at an earlier time point of the disease
wherein a significant decrease in the PSGR mRNA for said gene in the biofluid in comparison with the level of expression of said mRNA at the earlier time point is indicative that the prostate cancer shows a good progression or
wherein a significant increase or a lack of change in the PSGR mRNA for said gene in the biofluid in comparison with the level of expression of said mRNA at the earlier time point is indicative that the prostate cancer shows a bad progression.

6. A method according to claim 5 further comprising
(i) determining the levels of PCA3 mRNA in a biofluid from said subject,
(ii) comparing the levels obtained in step (i) and the levels obtained at an earlier time point with with predetermined cut off values for each of said genes wherein said predetermined cut off values for each gene correspond to the expression level of said gene which correlates with the highest specificity at a desired sensitivity in a ROC (receiver operating characteristics) curve calculated based on the expression levels of the PSGR and PCA3 genes determined in a patient population being at risk of suffering prostate cancer,
wherein a significant decrease in the expression level of at least one of said genes in the biofluid with respect to the expression level or levels at the earlier time point is indicative that the prostate cancer shows a good progression. wherein a significant increase or lack of change in the expression level of at least one of said genes in the biofluid with respect to the expression level or levels at the earlier time point is indicative that the prostate cancer shows a bad progression.

7. A method as defined in any of claims 2, 4, or 6 wherein the patient population being at risk of suffering prostate cancer is formed by patients having PSA levels above 4 ng/ml and/or patients with a positive DRE.

8. A method according to claim 7 wherein the patient population being at risk of suffering prostate cancer is formed by patients having PSA levels lower than 10 ng/ml.

9. A method according to any of claims 1 to 8 wherein the levels of PSGR mRNA and/or the levels PCA3 mRNA are normalized using the expression level of a housekeeping prostate gene.

10. A method according to claim 9 wherein the housekeeping prostate gene is PSA.

11. A method according to any of claims 1 to 10 wherein the biofluid is selected from the group or urine, blood, prostatic secretions and ejaculate.

12. A method according to claim 11 wherein the biofluid is urine obtained after a prostate massage.

13. A method according to claim 12 wherein the urine obtained after the prostate massage is the first voided urine.
